# EUROPEAN PATENT SPECIFICATION

(11) **EP 4 168 800 B1**
(45) Date of publication and mention of the grant of the patent: **30.09.2026**
(21) Application number: 21841866.3
(22) Date of filing: 16.07.2021
(51) Int. Cl.: G01N 33/493, A61B 10/00

(54) **URINE SAMPLE ANALYSIS SYSTEM**
URINPROBENANALYSESYSTEM
SYSTÈME D'ANALYSE D'ÉCHANTILLON D'URINE

(30) Priority: 16.07.2020 TR 202011323; 15.07.2021 TR 202111575
(43) Date of publication of application: 26.04.2023
(73) Proprietor: ORUBA MEDIKAL TEKNOLOJI ARGE IC VE DIS TIC. LTD. STI, 06800 Cankaya/Ankara (TR)
(72) Inventor: ULUDAG, Ahmet, 06800 Çankaya/Ankara (TR); DULGER, Ali Ogulcan, 06800 Çankaya/Ankara (TR); ULUSAHIN, Utku, 06800 Çankaya/Ankara (TR)
(74) Representative: Berkkam, Ayfer
(86) International application number: PCT/TR2021/050736
(87) International publication number: WO 2022/015276

(56) References cited:
- WO-A1-2017/193543
- WO-A1-2017/193543
- WO-A1-2020/139577
- CN-A- 108 130 943
- CN-A- 108 700 564
- CN-A- 109 283 324
- CN-U- 206 146 938
- CN-U- 207 114 549
- US-A1- 2018 372 717
- US-B2- 10 605 717

## Description

### Technical Field

This invention relates to a urine specimen analysis system that allows the chemical content to be analysed via camera and test strips in order for the current state of health of persons to be understood from their urine specimens.

### Prior Art

By performing content analysis of urine specimens, it is possible to diagnose many diseases or to diagnose the instant state of health. Absorbent surface to which reactive chemicals had been absorbed are used for the detection of chemical content. These absorbent surfaces change colours when the chemicals they contain react with the urine. By means of this colour change, the amounts of certain chemicals within the urine can be detected by the help of a scale.

The biomarkers the amount of which can be detected using the test strip in the urine are; proteins, glucose, ketone, haemoglobin, bilirubin, urobilinogen, acetone, nitrite and leukocytes. These biomarkers provide data to physicians so that they can diagnose many diseases. Therefore, tests on urine specimens are very often made in health institutions using the content analysis strips.

Today, there are patent applications related to automatic urine tests by means of strips placed inside toilets. One of these is described in United States patent applications no US1060571. In the said patent application, carrying out a urine analysis from the urine of the user by means of a plurality of strips placed under a water closer.

With mobile phones becoming widespread, carrying out this analysis anywhere with the help of a urine test strip and a mobile phone having a camera has become possible. One example of this is described in the patent application CN105223196. The camera of the mobile device detects the amount of biomarkers within the urine from the colour changes on the strip through an application, informs the user and can also save this information.

WO2020139577 discloses a urine analysis system comprising a fluid transportation system configured to expose at least one test strip to a urine sample. In addition, the device includes a sensor configured to capture an image of the at least one test strip exposed to the portion of the midstream urine sample.

US2018372717 discloses a system that collects a patient's urine, transports a portion to a test strip, and then cleans the collection component. A sensor images the wetted strip, a computer analyzes the image to determine patient condition, and a motor positions successive strips for testing and imaging.

### Brief Description of the Invention

This invention aims to realise a urine analysis system as defined in the independent claim and that carries on to the test strip in an amount that is required for the urine specimen to be analysed using the test strip. Another aim of the invention is to develop a urine analysis system that will ensure the chemical analysis of the urine by observing it with a camera after waiting for a period of time during which the urine strip will realise its chemical reactions. With its feature to clean itself and the test strips, the urine analysis system will be able to prevent both the previous and next users' urines to mix by means of cleaning the urine line and the system to stink.

### Detailed Description of the Invention

The urine analysis system realised for the invention to reach its aims was shown in the attached figures, among which;

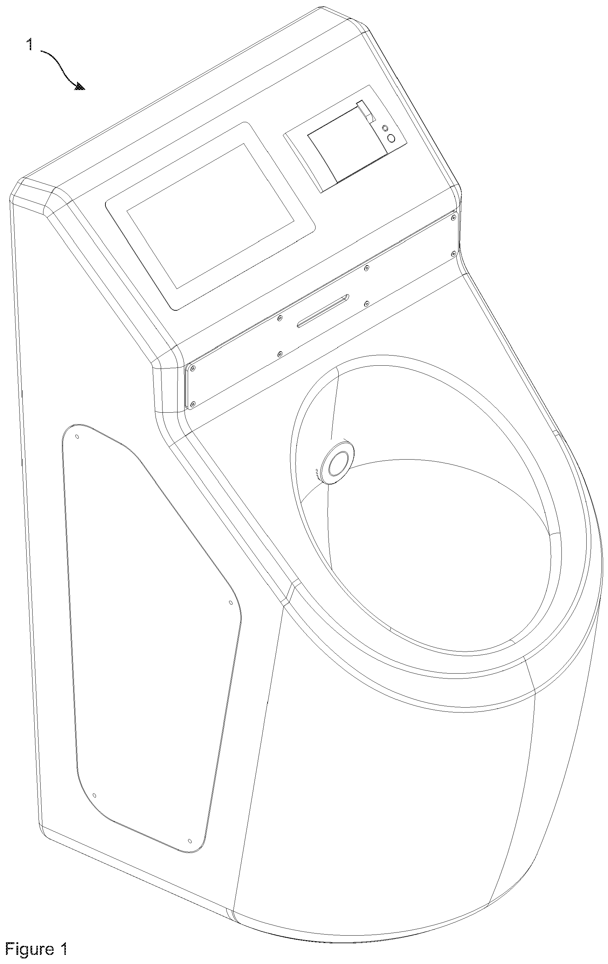

Figure 1 - Front view of the urinal embodiment of the urine specimen analysis system that is the subject of the invention.

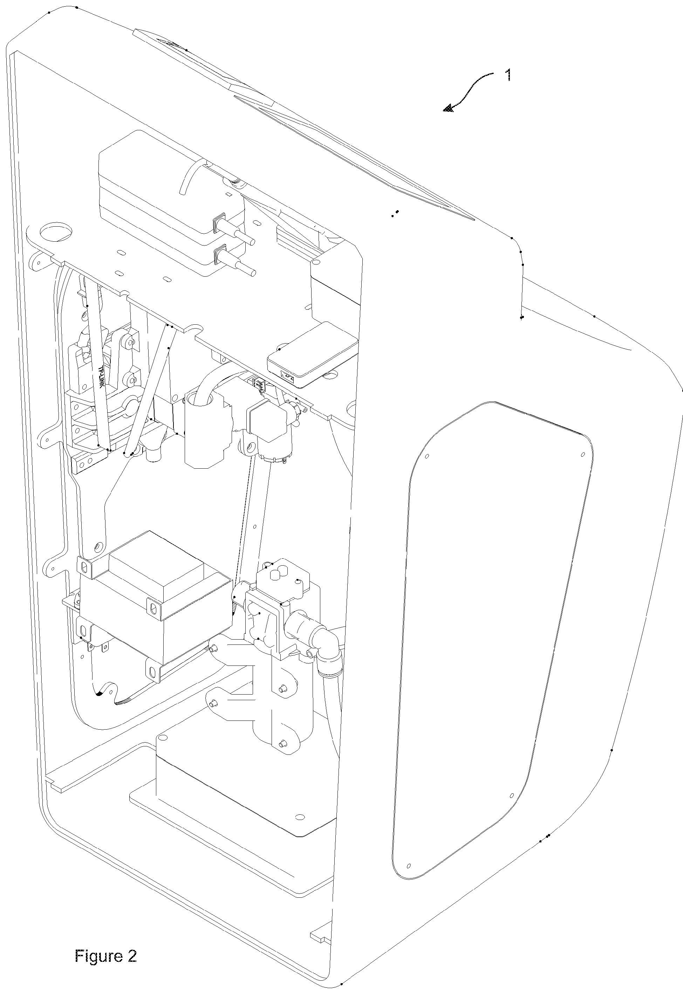

Figure 2 - Rear view of the urinal embodiment of the urine specimen analysis system that is the subject of the invention.

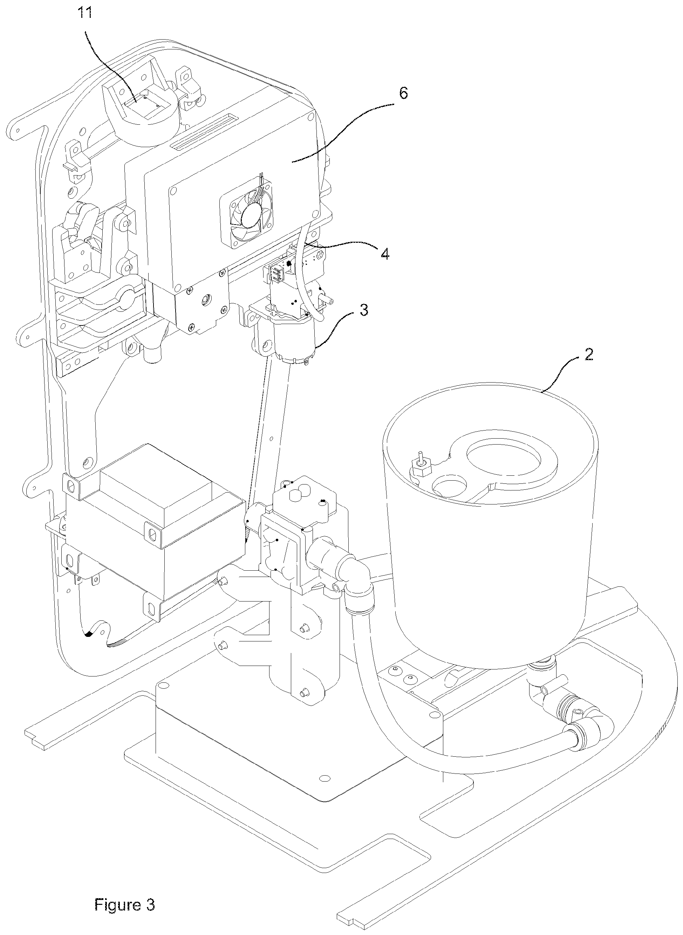

Figure 3 - Perspective view of the internal parts of the urinal or water closet of the urine specimen analysis system that is the subject of the invention.

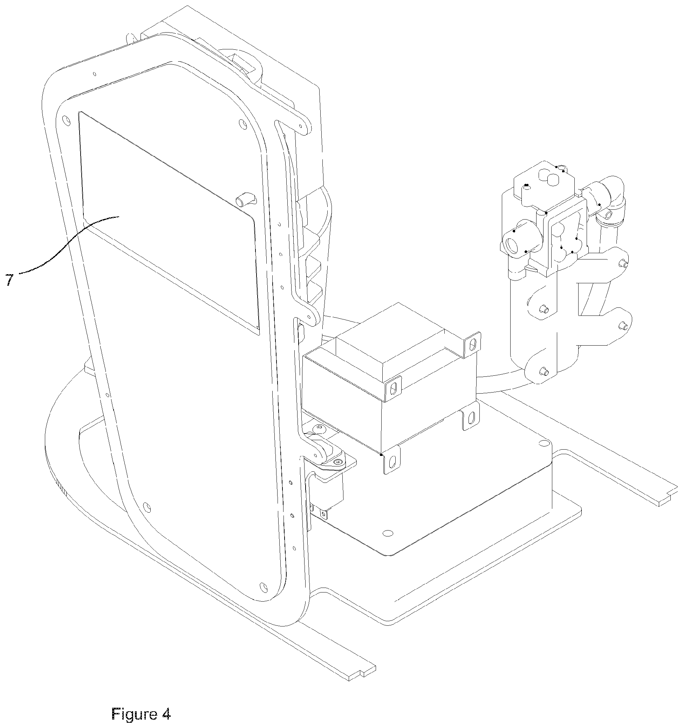

Figure 4 - Perspective view of the internal parts of the urinal or water closet of the urine specimen analysis system that is the subject of the invention, from the cassette cover side.

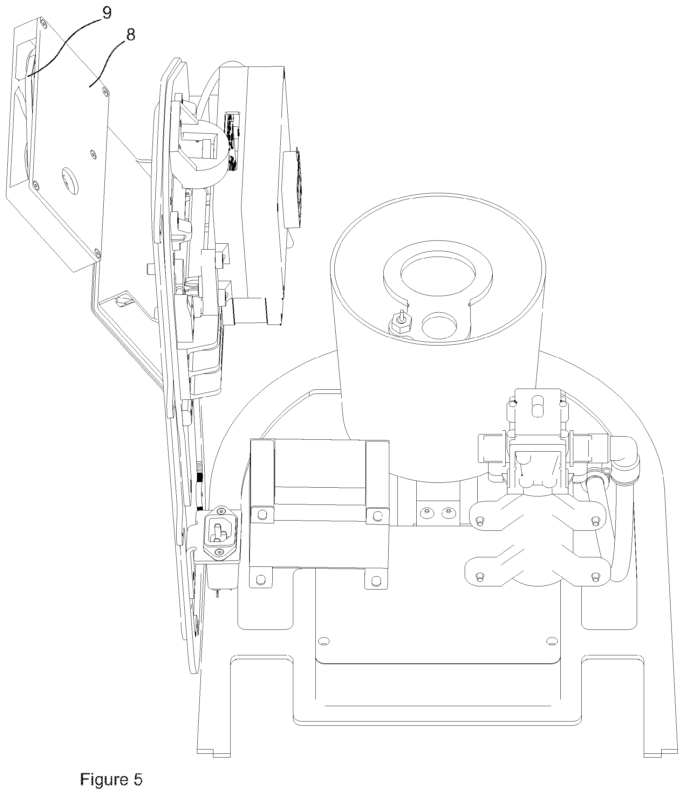

Figure 5 - Perspective view of the internal parts the urinal or water closet of the urine specimen analysis system that is the subject of the invention with the cassette cover open and cassette out.

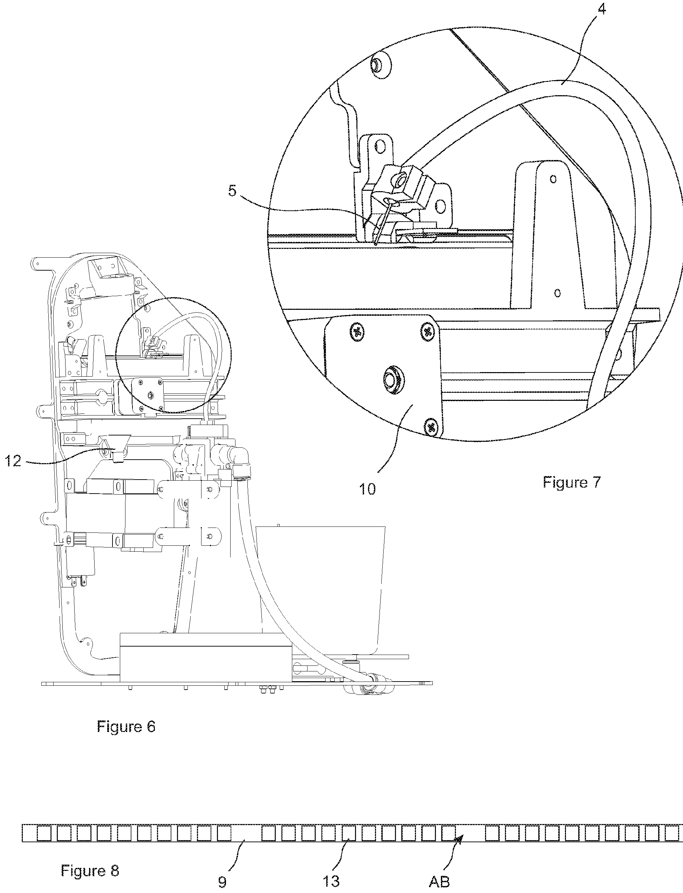

Figure 6 - Perspective view of the internal parts of the urinal or water closet of the urine specimen analysis system that is the subject of the invention, showing where the needle detail is taken.
Figure 7 - Perspective view of the internal parts of the urinal or water closet of the urine specimen analysis system that is the subject of the invention, showing the needle detail.
Figure 8 - View of an exemplary part of the test strip used in the urine specimen analysis system that is the subject of the invention.

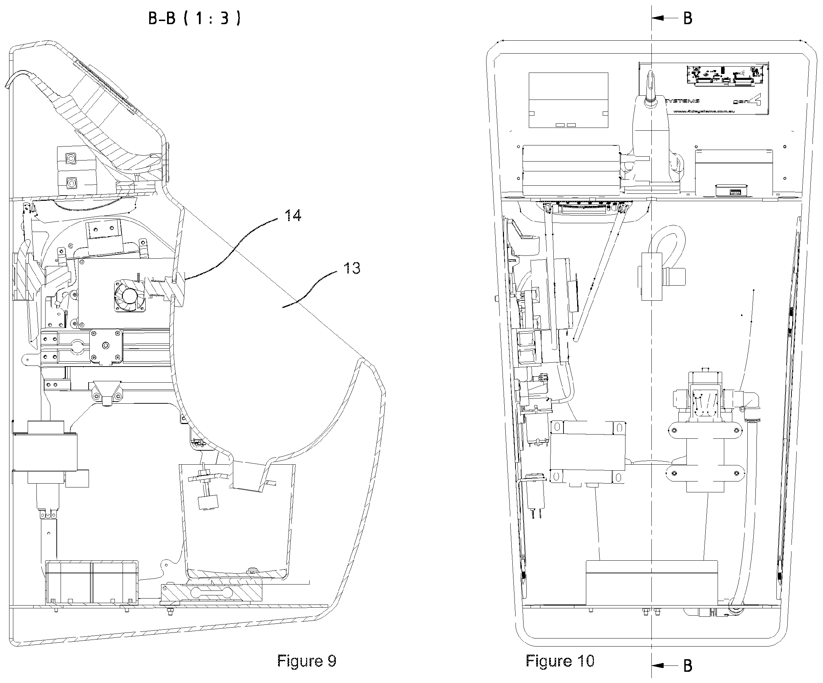

Figure 9 - B-B sectional view of the urine specimen analysis system that is the subject of the invention, mounted within the urinal.
Figure 10 - Front view showing the axis where the B-B sectional view of the urine specimen analysis system that is the subject of the invention, mounted within the urinal, is taken.

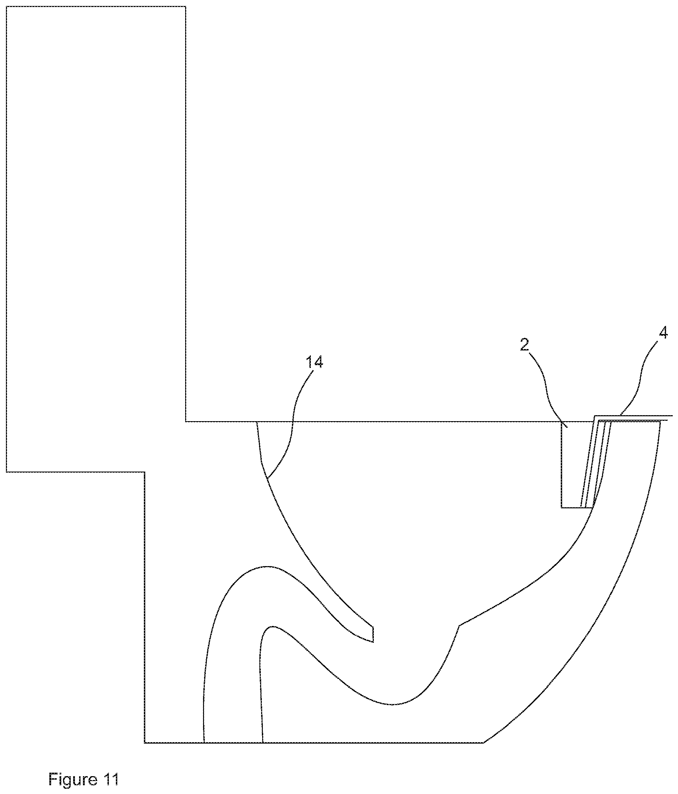

Figure 11 - Schematic view with a sectional line taken from the middle of the urine specimen analysis system that is the subject of the invention, mounted within the water closet.
Sekil 12 - Schematic perspective view of the urine specimen analysis system that is the subject of the invention comprising needle and vacuum line.

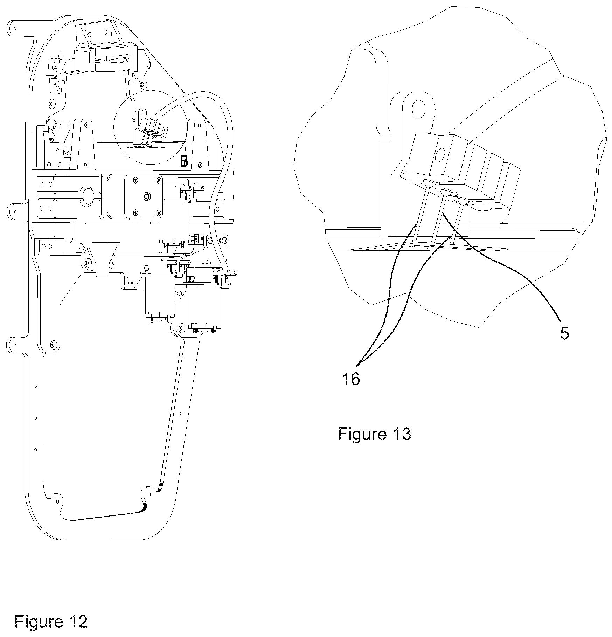

Figure 13 - Detailed schematic perspective view of the urine specimen analysis system that is the subject of the invention comprising the needle and vacuum line.

The parts in the figures are enumerated and the parts the numbers refer to are given below.
1. Urine Specimen Analysis System
2. Collection container
3. Engine
4. Urine feed line
5. Needle
6. Control unit
7. Cassette cover
8. Cassette
9. Test strip
10. Cassette motor
11. Camera
12. Urine collection funnel
13. Absorbent layer
14. Bowl
15. Water outlet
16. Vacuum line

Urine specimen analysis system (1) that is the subject of the invention, in its simplest form, comprises a water closet or a urinal bowl/body into which urine specimen can be given, test strip (9), camera (11) and a pump engine (3) and needle (5) that carry the urine to the test strip (9). The needle (5) ensures that urine is carried on to the test strip (9) directly in sufficient amount.

By the help of the urine reaching on the absorbent layers (13) on the test strip (9) sufficient enough for the reaction, the colour change on the layers will be observed. Additionally, as sufficient amount of urine will be taken from the needle (5) the amount of urine that will stink in the system will be reduced.

The test strip (9) shall move linearly in front of the needle (5) for the test to be performed. The speed and amount of the movement of the test strip (9) varies depending on the total number of layers (13) on the test strip (9) onto which the test chemical was absorbed. While it is enough for the test strip (9) to move as long as the urine absorbance region to perform 3 tests in front of the needle (5) in the case of testing for 3 different chemicals, the amount of movement will cover regions for 10 test on a test strip (9) comprising 10 tests. In addition to that, to prevent each urine flow from polluting the next test region on the test strip (9), there is a certain spacing (S) between each test strip (9) section.

The process of advancing the test strip (9) as far as all the test absorbent layer (13) surfaces and the spacing itself is carried out by a cassette motor (10). Because while the test strip (9) is wrapped in a cassette (8), the urine sample can be inserted in and removed from the urine specimen analysis system (1). The cassette (8) is the most suitable form for storing, reading, writing of a strip material and moving any strip inside it. For this reason, a cassette cover (7) is attached to the body of the urine specimen analysis system (1) and the cassette (8) can be attached and removed by means of it. The cassette motor (10), which will enable the test strip (9) to be rotated by attaching the cassette (8) in place, enters into the slot where it will move the reel of the cassette (8).

The amount of movement and speed of the cassette motor (10) is determined according to the operational speed of the pump motor (3) and the amount of urine coming from the tip of the needle (5). If the cassette motor (10) moves fast, enough urine may not reach the absorbent layers (13). In the contrary case, it is possible that the excess urine will fill into the cassette (8) and pollute it in a way to stink.

The method of embodiment of the urine specimen analysis system (1), that is the subject of the invention, shown in the attached figures, includes a component group that can be mounted inside a urinal or a water closet or on the wall. The most important element in this component group is the collection container (2) in which the urine is collected. The collection container (2) is placed under the bowl (14) for the urinal embodiment shown in the figures.

In water closet bowl type embodiments, a collection container (2) can be placed close to the front of the closet, not under it, to collect the urine specimen so that solid faeces does not mix with the urine. It is possible for a urine feed line (4) coming out of this collection container (2) to suck the urine with the suction of the pump motor (3) and deliver it to the test strip (9).

Whether in urinal or water closet bowl embodiment, the whole process will be carried out by a control unit (6). Entire process including telling the users that the system is ready for the test and they can start urinating, and the process of sucking the urine with the feed line (4) when it is collected in the collection container (2) and delivering it to the test strip (9) will be controlled by the control unit (6). The chemicals contained in the absorbent layers (13) change colour over a period of time when they encounter urine. This time varies between 30 seconds and 120 seconds depending on the chemical entering the reaction. During this period, the waiting and observing the amount of change with the camera (11) is also tracked by the control unit (6).

Cleaning the test strip (9) and absorbent layers (13) after each test run is a necessity to avoid stinking. For this purpose, own water outlet (15) system of the urinal or water closet will be used. Generally, the high pressure water provided by this water outlet (15) can ensure the cleaning of the entire surface of the bowl (14) to which the urine had contacted. In this way, by means of the water that can also reach the collection container (2), it is possible to supply water to the entire urine feed line (4) and to deliver this water up to the test strip (9). The test strip (9) will be washed with a large amount of water and will be purified from urine residues.

At the end of each process, first of all, the needle (5) and the feed line (4), which are emptied by vacuum instead of spraying, will be cleaned. Discharging of the water sent on the test strip (9) and the urine residues after that is important for the stable operation of the system. For this reason, there is an opening under the cassette (8) for the discharging of the urine and water filled into the cassette (8). Water and urine residues coming off this opening are collected by the urine collection funnel (12) and sent to the drain.

The total number of tests inside the test strip (9), the length of the strip, the length and number of the separating partitions (S) separating the absorbent layer (13) groups from one another are predefined in the control unit (6). In addition to that, the visual data provided by the camera (11) and the number of rotation of the cassette motor (10) will be used in the continuous control and calibration of these values.

The amount of urine delivered onto the test strip (9) by means of the needle (5) may be higher than the amount of absorption of the absorbent layers (13), and it is possible for urine droplets to remain on the test strip (9) apart from the absorbent layers (13). These urine droplets both cause stinking and the images taken by the camera (11) to deteriorate. Urine droplets should be collected from over the test strip (9) as a precaution against both problems.

On the other hand, it is possible for the urine coming from the needle (5) to flow in large amounts and suddenly due to a problem similar to the sudden clearance of an obstruction. In this case, to prevent the pollution of the test strips (9) that are kept as clean rolls, the excess urine should be extracted not to contaminate the other strips.

The vacuum line (16) will be used to collect excess urine from the test strip (9) area on which the needle (5) drips urine. The vacuum line (16) will ensure that the excess urine that the absorbent layers (13) do not absorb will be removed from the front, back, right, left and even from the entire circumference of the needle (5) angularly by means of one or more vacuum pumps. The proximity of the vacuum line (16) to the needle (5), the vacuum power, the frequency and sequence of operation can be adjusted to ensure that no urine drops are left on the test strip (9).

For the camera (11) to easily detect colour changes on the test strip (9), a printed image containing reference colours can be placed in a region within the camera's (11) field of view. By this way, as the ambient lighting will illuminate both the test strip (9) and the printed images at the same time, the measurement will be made without being affected by the wavelength changes.

In a different embodiment of the urine specimen analysis system (1) that is the subject of the invention, instead of taking the urine to be used to treat the absorbent layers (13) with urine to perform the analysis from the collection container (2) via the urine feed line (4), it is possible to feed it by means of a feed line (4) from the urine accumulating in the bowl (14) during urination or from a drain line directed from the bowl (14). In this embodiment of the invention, although there is no accumulation of urine in a reservoir, it will be possible to collect urine from any line during the transfer of urine from the bowl (14) to the drain.

It is possible for the test strip layout, which can be designed in different geometric shapes, to be placed in a closed field. In this case, the thing that is tried to be explained is to include test strips (9) to be used in a closed environment that is desired to be told with the use of cassettes (8).

Again, the camera (11) positioned to face the test strip (9) directly or with different optical means and is to be used in the urine specimen analysis system (1) that is also the subject of the invention, can be used as a colour sensor or image sensor or wavelength sensor.

In water closet type embodiments, it is possible to use a separator/sorter that separates solid and liquid faeces from each other instead of the bowl (14). By means of the separator/sorter used during this embodiment, it is possible to ensure that the urine remains in the front of the separator in accordance with human ergonomics, and direct the solid faeces to the rear side in accordance with the same female and male ergonomics. Due to the nature of body ergonomics of a man or woman in a sitting position is expected to urinate forward and defecate backwards/to the ground. Accordingly, the separator/sorter that separates the incoming faeces into two parts, will be able to direct urine and faeces to different places with its two funnel structures.

## Claims

1. A urine specimen analysis system (1) configured to determine the current state of health of persons from the chemical content of urine specimens, comprising:
- a test strip (9) comprising an absorbent layer (13) within a cassette (8),
- a camera (11) facing the test strip (9),
- a needle (5) adapted to deliver the urine to the test strip (9);
- a pump motor (3) adapted to pump the urine to the needle (5);
- a urine feed line (4) that directs the urine to the test strip (9) from accumulation in the bowl (14) or from a drain line directed from the bowl (14) for urine to be delivered to the absorbent layer (13) of the test strip (9).
**characterized by**;
- a cassette motor (10), configured to move the test strip (9) in front of the needle (5), wherein the amount of movement and speed of the cassette motor (10) are determined based on the operational speed of the pump motor (2) and the amount of urine coming from the tip of the needle (5).

2. A urine specimen analysis system (1) of Claim 1, **characterized by** using vacuum instead of spraying to clean the urine remaining in the needle (5) and the feed line (4).

3. A urine specimen analysis system (1) of Claims 1-2, **characterized by** the discharge of the water and urine remains on the test strips (9) as well.

4. A urine specimen analysis system (1) of Claims 1-3, **characterized by** a urine collection funnel (12) through which the remains dropping out from an opening under the cassette (8) for discharging the urine and water filling into cassette (8) are collected and discharged.

5. A urine specimen analysis system (1) of Claims 1-4, **characterized by** a vacuum line (16) ensuring that the excess urine that the absorbent layers (13) do not absorb will be removed from the front, back, right, left and the entire circumference of the needle (5) angularly by means of one or more vacuum pumps for collecting the excess urine from the test strip (9) area where the needle (5) drips the urine.

6. A urine specimen analysis system (1) of Claims 1-5, **characterized by** a collection container (2) to collect the urine specimen from an area close to the front side of the water close to ensure that solid faeces does not mix with the urine.

7. A urine specimen analysis system (1) of Claims 1-6, **characterized by** a separator/sorter that ensures the separation of solid and liquid faeces in the water closet and can direct these liquid and solid faeces to different directions by means of two funnel structures during urination to the front and defecation to the backwards/to the ground in the sitting position due to their ergonomics.

## Patentansprüche

1. Urinprobenanalysesystem (1), das dazu eingerichtet ist, den aktuellen Gesundheitszustand von Personen aus dem chemischen Gehalt von Urinproben zu bestimmen, umfassend:
- einen Teststreifen (9) umfassend eine Absorptionsschicht (13) innerhalb einer Kassette (8);
- eine Kamera (11), die dem Teststreifen (9) zugewandt ist;
- eine Nadel (5), die dazu ausgebildet ist, den Urin dem Teststreifen (9) zuzuführen;
- einen Pumpenmotor (3), der dazu ausgebildet ist, den Urin zu der Nadel (5) zu pumpen;
- eine Urinzuführleitung (4), die den Urin aus einer Ansammlung in dem Becken (14) oder aus einer von dem Becken (14) abgeleiteten Ablaufleitung zu dem Teststreifen (9) leitet, damit der Urin der Absorptionsschicht (13) des Teststreifens (9) zugeführt wird.
**gekennzeichnet durch**;
- einen Kassettenmotor (10), der dazu eingerichtet ist, den Teststreifen (9) vor die Nadel (5) zu bewegen, wobei das Bewegungsausmaß und die Geschwindigkeit des Kassettenmotors (10) auf der Grundlage der Betriebsgeschwindigkeit des Pumpenmotors (2) und der Urinmenge, die aus der Spitze der Nadel (5) kommt, bestimmt werden.

2. Urinprobenanalysesystem (1) nach Anspruch 1, **gekennzeichnet durch** die Verwendung von Vakuum anstelle von Sprühen, um den in der Nadel (5) und der Zuführleitung (4) verbleibenden Urin zu reinigen.

3. Urinprobenanalysesystem (1) nach einem der Ansprüche 1 bis 2, **gekennzeichnet durch** das Ableiten des Wassers und der Urinreste auf den Teststreifen (9) ebenfalls.

4. Urinprobenanalysesystem (1) nach einem der Ansprüche 1 bis 3, **gekennzeichnet durch** einen Urinauffangtrichter (12), durch den die Reste, die aus einer Öffnung unter der Kassette (8) zum Ableiten des Urins und des in die Kassette (8) einlaufenden Wassers herausfallen, aufgefangen und abgeleitet werden.

5. Urinprobenanalysesystem (1) nach einem der Ansprüche 1 bis 4, **gekennzeichnet durch** eine Vakuumleitung (16), die sicherstellt, dass der überschüssige Urin, den die Absorptionsschichten (13) nicht absorbieren, von vorne, von hinten, von rechts, von links und von dem gesamten Umfang der Nadel (5) winklig mittels einer oder mehrerer Vakuumpumpen entfernt wird, um den überschüssigen Urin aus dem Bereich des Teststreifens (9) aufzufangen, in dem die Nadel (5) den Urin abtropft.

6. Urinprobenanalysesystem (1) nach einem der Ansprüche 1 bis 5, **gekennzeichnet durch** einen Auffangbehälter (2) zum Auffangen der Urinprobe aus einem Bereich in der Nähe der Vorderseite des Wasserklosetts, um sicherzustellen, dass die festen Fäkalien sich nicht mit dem Urin vermischen.

7. Urinprobenanalysesystem (1) nach einem der Ansprüche 1 bis 6, **gekennzeichnet durch** einen Trenner/Sortierer, der die Trennung von festen und flüssigen Fäkalien in dem Wasserklosett sicherstellt und diese flüssigen und festen Fäkalien mittels zweier Trichterstrukturen in verschiedene Richtungen leiten kann, während des Urinierens nach vorne und des Defäkierens nach hinten/auf den Boden in der sitzenden Position aufgrund ihrer Ergonomie.

## Revendications

1. Système d'analyse d'échantillon d'urine (1) configuré pour déterminer l'état de santé actuel de personnes à partir du contenu chimique d'échantillons d'urine, comprenant:
- une bandelette de test (9) comprenant une couche absorbante (13) à l'intérieur d'une cassette (8);
- une caméra (11) faisant face à la bandelette de test (9);
- une aiguille (5) adaptée pour acheminer l'urine vers la bandelette de test (9);
- un moteur de pompe (3) adapté pour pomper l'urine vers l'aiguille (5);
- une conduite d'alimentation en urine (4) qui dirige l'urine vers la bandelette de test (9) depuis une accumulation dans la cuvette (14) ou depuis une conduite d'évacuation dirigée depuis la cuvette (14), afin que l'urine soit acheminée vers la couche absorbante (13) de la bandelette de test (9).
**caractérisé par**;
- un moteur de cassette (10), configuré pour déplacer la bandelette de test (9) devant l'aiguille (5), dans lequel l'amplitude du mouvement et la vitesse du moteur de cassette (10) sont déterminées en fonction de la vitesse de fonctionnement du moteur de pompe (2) et de la quantité d'urine provenant de la pointe de l'aiguille (5).

2. Système d'analyse d'échantillon d'urine (1) selon la revendication 1, **caractérisé par** l'utilisation d'un vide au lieu d'une pulvérisation pour nettoyer l'urine restant dans l'aiguille (5) et la conduite d'alimentation (4).

3. Système d'analyse d'échantillon d'urine (1) selon l'une quelconque des revendications 1 à 2, **caractérisé par** l'évacuation de l'eau et des restes d'urine sur les bandelettes de test (9) également.

4. Système d'analyse d'échantillon d'urine (1) selon l'une quelconque des revendications 1 à 3, **caractérisé par** un entonnoir de collecte d'urine (12) à travers lequel les restes tombant d'une ouverture située sous la cassette (8) pour l'évacuation de l'urine et de l'eau se remplissant dans la cassette (8) sont collectés et évacués.

5. Système d'analyse d'échantillon d'urine (1) selon l'une quelconque des revendications 1 à 4, **caractérisé par** une conduite de vide (16) assurant que l'urine excédentaire que les couches absorbantes (13) n'absorbent pas sera retirée de l'avant, de l'arrière, de la droite, de la gauche et de toute la circonférence de l'aiguille (5) de manière angulaire au moyen d'une ou plusieurs pompes à vide pour collecter l'urine excédentaire de la zone de la bandelette de test (9) où l'aiguille (5) égoutte l'urine.

6. Système d'analyse d'échantillon d'urine (1) selon l'une quelconque des revendications 1 à 5, **caractérisé par** un récipient de collecte (2) destiné à collecter l'échantillon d'urine depuis une zone proche du côté avant des toilettes afin d'assurer que les matières fécales solides ne se mélangent pas avec l'urine.

7. Système d'analyse d'échantillon d'urine (1) selon l'une quelconque des revendications 1 à 6, **caractérisé par** un séparateur/trieur qui assure la séparation des matières fécales solides et liquides dans les toilettes et qui peut diriger ces matières fécales liquides et solides vers des directions différentes au moyen de deux structures d'entonnoir, lors de la miction vers l'avant et de la défécation vers l'arrière/vers le sol en position assise en raison de leur ergonomie.
